# EUROPEAN PATENT SPECIFICATION

(11) **EP 4 525 695 B1**
(45) Date of publication and mention of the grant of the patent: **28.10.2026**
(21) Application number: 23727704.1
(22) Date of filing: 08.05.2023
(51) Int. Cl.: A61B 5/00, A61B 5/11

(54) **UNOBTRUSIVE METHOD AND DEVICE FOR SEIZURE DETECTION**
UNAUFFÄLLIGES VERFAHREN UND VORRICHTUNG ZUR ERKENNUNG VON KRAMPFANFÄLLEN
PROCÉDÉ ET DISPOSITIF DISCRET DE DÉTECTION DE CRISE

(30) Priority: 17.05.2022 PT 2022117985
(43) Date of publication of application: 26.03.2025
(73) Proprietor: Instituto Superior Técnico, 1049-001 Lisboa (PT); Instituto de Telecomunicações, 3810-193 Aveiro (PT)
(72) Inventor: GARÇÃO, Vicente, 1049-001 Lisboa (PT); MARTINS, Rita, 1049-001 Lisboa (PT); BALTAZAR, Ana Filipa, 1049-001 Lisboa (PT); PATA, Kihara Sofia, 1049-001 Lisboa (PT); AZEITONA, António, 1049-001 Lisboa (PT); ABREU, Mariana, 1049-001 Lisboa (PT); FRED, Ana Luisa, 1049-001 Lisboa (PT); SILVA, Hugo, Lisboa (PT)
(74) Representative: Pereira da Cruz, Joao
(86) International application number: PCT/PT2023/050011
(87) International publication number: WO 2023/224502

(56) References cited:
- EP-A2- 2 123 221
- US-A1- 2016 302 714
- US-A1- 2021 100 492
- US-B1- 10 271 019

## Description

### Technical field of the invention

This invention is related to the fields of biosignal monitoring, epileptic seizure detection, biomedical motion recognition and computer vision.

### State-of-the-art

Epilepsy is a neurological condition characterized by the repeated occurrence of unprovoked seizures which affects 50 million people worldwide. Seizures are periods of abnormal, uncontrolled, and excessive electrical activity in the brain that disrupt brain function and can cause motor symptoms, altered sensory experiences and loss of consciousness.

Seizure detection devices can have multiple benefits for patients with conditions such as epilepsy and their caregivers, such as decreasing stress and anxiety levels, and helping more objectively log seizures and sharing this information with health professionals. According to patient surveys [1], these devices needed to be comfortable and unobtrusive enough as to not interfere with either patients' sleeping quality or their daily activities. Accuracy, affordability, data confidentiality and privacy were also significant worries.

Many studies have developed seizure detection methods and devices. According to review studies [2], seizure detection modalities include EEG, accelerometry (ACM), sEMG, ECG, and video detection, among others. Most of these methods have the significant disadvantage of requiring the user to continually wear a device that may be obtrusive or uncomfortable, or may remind them or others of their condition, potentially leading to situations where the user is stigmatized. Video-based seizure detection methods, especially those that do not require markers, are unobtrusive by nature, in that they only require a device with a video camera, and do not need to be worn by the user. Therefore, video-based seizure detection has been studied as an unobtrusive alternative to existing seizure detection methods. Other video-based methods use markers to highlight body parts and calculate movement. However, marker-based approaches have the disadvantage of not being very practical, due to markers possibly being occluded by blankets, for example.

Most marker-less methods in the state of the art calculate motion features using Optical Flow (OF). A marker-less method disclosed in [3] calculated motion parameters from OF and classified these based on "spectral contrast", a measure of the power within the 2-6 Hz frequency range.

Another study [4] devised an OF-based algorithm for the detection of neonatal seizures used a feed-forward neural network and reported a sensitivity and specificity between 82% and 95%. A separate study [5] used deep learning networks and reported a sensitivity of 88%, a specificity of 92%, and a latency of 22 seconds. Video-based seizure detection methods published in literature have shown good accuracy. US10595766B2 describes a system and method for detecting seizures using video and accelerometry data, possibly in tandem. US10504226B2 outlines a seizure detection system and method using one or more 3D cameras. Further, EP2123221 discloses a seizure detection system comprising video cameras, accelerometers, motion detectors processors to analyze the signals from video camera and/or motion detectors, or hybrid setups. US2016/302714 discloses a seizure detection system comprising a video capture device to capture motion of a patient. US2021/100492 also discloses a seizure monitoring system and device comprising a video camera.

Video-based seizure detection has many advantages, such as not being uncomfortable, providing remote monitoring for caregivers or enabling accurate seizure registration. Nevertheless, it is essential to ensure that such a system is unobtrusive and privacy preserving. The concept of "invisibles" has arisen in recent years regarding unnoticeable devices that can be seamlessly integrated into a user's life [6]. An example of such a device is "Emerald", a discreet device for monitoring patients with Parkinson's Disease that monitors sleep stages and gait patterns using radio waves, as described in [7]. Invisibles can facilitate physiological monitoring and enable improvements in quality of life and diagnostic accuracy for patients with conditions like epilepsy, without negatively affecting their day-today life or exposing their medical condition to others, which could lead to stigma [8].

Integrating a video-based seizure detection device within daily objects, such that it is nearly invisible, would ensure that this device works unobtrusively and does not remind the user or others of their condition. An example of an integration of a similar system, in this case for video surveillance purposes, within a household object, is described in US10587846B1 which consists of a camera system integrated within a lightbulb which transmits data via power-line communication.

### Summary of the invention

This invention is related to the field of epileptic seizure detection in that it consists of a device designed to detect epileptic seizures and alert caregivers or health professionals. In this sense, it is related to biosignal monitoring and biomedical motion recognition in that it continually records and processes biosignals that correspond to body movements and detects patterns that correspond to seizures. The use of video recordings connects this invention to the computer vision field.

More specifically, the present invention refers to a device comprised by a computational unit (200), that is integrated within an enclosure (100), and which is connected to a video camera unit (500), to a regular and to an infrared lighting module (601 and 602, respectively), to a control module (300) and to a transmission module (400).

The device is aimed at detecting and recognizing seizures or other movements of interest, issuing automated alarms, registering the time, length, and frequency of seizures or occurrences of interest as well as other statistical information for the user, and facilitating the sharing of data with health professionals if this option is chosen by the user.

The device possesses two key characteristics, namely the intrinsic preservation of user privacy and its "invisible" nature, defined by the fact that the device is enclosed such that it is not obtrusive, cumbersome, or immediately noticeable, blending in with the user's home environment. This ensures the device does not remind the user or others of the user's condition and does not negatively impact the user's daily life.

### Computational Unit

The computational unit (200) can be any electronic equipment suitable for the processing of video footage and compatible with the digital signal processing performed by the seizure detection method developed. Its mode of functioning is determined by the accompanying seizure detection method. The computational unit (200) must be capable of interfacing with, receiving input from, powering, and/or controlling a control module (300) and a transmission module (400). Furthermore, it must be capable of powering, controlling, and/or receiving input from a video camera module (500) and regular and infrared lighting modules (601 and 602).

The computational unit (200) must be capable of processing video footage captured by the camera module (500). The processing of the video footage includes the calculation of Optical Flow which is a motion detection, quantification or recognition method, and the digital signal processing also includes the application of an independent component separation technique, namely Independent Component Analysis (ICA), followed by Machine Learning or Thresholding Classification Methods. Alternatively, the digital signal processing may include the application of a dimensionality reduction technique such as Principal Component Analysis (PCA), after the calculation of the OF and before the execution of the independent component separation technique.

The computational unit (200) may be composed of one or more units, which could be present inside or outside the unobtrusive enclosure. One possible embodiment would be the use of a microcontroller such as, for example, an Arduino, ESP32, or Raspberry Pi Pico device of small dimensions, for the purpose of powering and controlling the aforementioned modules and receiving and transmitting inputs such as video footage or Optical Flow signals, which would then be processed by an external device such as a Raspberry Pi 4, a laptop computer or smartphone, or another such device. This processing would then determine whether an occurrence of interest was occurring and transmit this information or issue an alarm.

Another embodiment would be the integration of a computational unit that would power and control the aforementioned modules, receive a video footage input, perform all processing steps, and transmit information about possible occurrences and/or video data.

### Control Module

The computational unit (200) must be capable of interfacing with a control module (300), which may exist in the form of tactile buttons, a touch screen which may be capacitive or resistive and may contain multi-touch functionality, a mobile application, or other user interface methods. This control module (300) may enable the user to control when the device should start or stop recording videos, the type of occurrence being detected, which data should be stored locally or remotely, the mode of functioning of the device, and other functionalities.

### Transmission Module

The transmission module (400) must be able to receive information regarding possible occurrences or video data from the computational unit (200), and transmit it to the user, a caregiver, or a remote database. The data to be transmitted may be a report of the detected occurrences, a segmented video file with video footage from just the detected occurrence, or video data from long recorded time periods. In an embodiment, the transmission module (400) may consist of a power-line communication (PLC) module which may transmit the data through the electrical grid. This may be chosen in an embodiment where the device is enclosed within a lightbulb, foregoing the need for Bluetooth or wi-fi communications.

In another embodiment, this transmission module (400) may be integrated within the computational unit (200), and the data transmission may use a mobile data, wi-fi, Bluetooth, or other type of wired or wireless connection. This may take advantage of the capabilities of Raspberry Pi or ESP32 devices.

### Detailed Description of the Invention

The proposed invention allies the concept of "invisibles" with the needs of epilepsy patients. Not only does this invention overcome several limitations of current seizure detection devices in an inventive and innovative manner, but it also surpasses many of the challenges of wearable health monitoring technology, by providing highly accurate detection of seizures due to its innovative seizure detection method, while being unobtrusive, unnoticeable, preserving privacy by design, and providing live video footage and automated alarms for caregivers when dangerous seizure episodes occur. The use of a single hidden video camera encased in a light fixture, household object or another discreet enclosure provides a significant advantage over regular video-based seizure detection, and the use of a marker-less video modality ensures unobtrusive and contactless seizure detection.

Regarding the novel seizure detection method, the combination of Optical Flow (OF), Independent Component Analysis (ICA) and Machine Learning (ML) Classification is not only novel, but also provides distinct advantages regarding seizure detection. Optical Flow generates unidentifiable representations of the video data, enabling the device to not need to store video footage locally or remotely at all, if the user so desires, ensuring privacy is preserved by design. Moreover, the use of ICA to isolate seizure movement, when coupled with a source selection step, ensures that seizure movements are separated from not seizure movement, meaning that this device detects seizures even when other people are moving in front of the person having a seizure. This is a significant advantage over the state of the art because it enables daytime seizure detection with fewer false alarms.

The device comprises a computational unit (200) in an unobtrusive enclosure (100), that records video through a video camera unit (500) and processes it. This computational unit (200) may be any sort of electronic processing device that has processing capabilities adapted to implement the seizure detection method and alters the device's functioning, behavior, or properties. The computational unit (200), as mentioned, may consist of more than one electronic device, as long as it interfaces, communicates with, or powers the different modules and implements the method which may be described as follows.

In an embodiment, a single-board computer such as a Raspberry Pi device or an analogous device may be included into the enclosure (100) to perform all the necessary processing steps, as well as control the different modules. This electronic device would need to possess the necessary computational power to execute the method that defines the functionality of the device. In such an embodiment, the integrated communication capabilities of such a device may constitute the transmission module (400), by using communication protocols such as Bluetooth, Near-field communication (NFC), Wi-fi, or other types of data transmission. The data, which may constitute information or video data, may be stored on the device, on a remote database, or on another device, such as the user's smartphone or laptop computer. If a computational unit (200) with a General-Purpose Pinout (GPIO) is employed, this may be used to implement the control module (300) as buttons or as a touch screen display placed on the enclosure (100). Additionally, the use of a single-board computer as a computational unit (200) may facilitate the addition of a small form factor display to the enclosure (100). This display would be powered by the computational unit (200) and would have a retractable mechanism in order to ensure unobtrusiveness, remaining hidden when not in use. It may be used to help line up the camera's field of view with the user's bed or position.

In an embodiment that would ensure the device is compact and/or portable, the computational unit (200) would be a small form factor microcontroller. This unit (200) could be an Arduino device, an ESP32 device, a Raspberry Pi Pico or Zero, or another such device. This microcontroller could power and control the lighting modules (601 and 602), receive a video feed from the video camera unit (500), and, if computationally capable, compute Optical Flow and transmit the Optical Flow vectors via the transmission module (400). If this unit (200) is not computationally capable to compute Optical Flow in real-time, it may be used only to receive and transmit the video feed from the camera module (500) to an external computational unit (200), which would be tasked with executing the detection method and issuing alarms. This could be a single-board computer or another device, such as the user's smartphone, a tablet, laptop or desktop computer or another similar device. In such an embodiment, the small form factor of the microcontroller would enable the enclosure to be compact, such as a light bulb. In this case, power-line communication (PLC) could be used to transmit data to the larger and more computationally capable processing unit, which could be connected to an electrical socket on the wall. Alternatively, a wireless communication protocol such as Wi-Fi or Bluetooth could be used to transmit the data to a device such as the user's smartphone, a tablet, laptop or desktop computer or another similar device.

Video footage is recorded by the video camera unit (500) and transmitted to the computational unit (200). A Region-Of-Interest (ROI) within the video frame that isolates the movements made by the user from other movements is automatically selected. A motion recognition, quantification or detection method then processes the video footage. Particularly, Optical Flow is used, which detects movement velocities from the apparent brightness patterns of a video sequence, at equidistant or feature-based points in the video frame. This has the advantage of generating an unidentifiable representation of the movement data, preserving user privacy by design. Different implementations of Optical Flow can be used, including but not limited to the Farnebäck two-frame motion estimation method, which is the preferred method, since it is based on only two consecutive frames, enabling it to be calculated in real time and on a small form factor processing device such as a Raspberry Pi. Optical Flow is the preferred motion recognition, quantification, or detection technique, but others may be employed, including but not limited to deep-learning Optical Flow methods, Convolutional Neural Networks (CNN) or other deep-learning methods, frame differences, pose estimation, or person detection.

Optical Flow enables the calculation of the magnitude, direction, and frequency of movements. The signals recovered from Optical Flow, namely the movement velocities at equidistant or feature-based points in the video frame, are computed continually over time to generate time-series signals. Video recording is stopped and restarted at regular time intervals, between 5 seconds and 2 minutes ideally, whereby the acquired video footage over the last segment is processed using Optical Flow, generating time-series signals. The Optical Flow movement vectors may be split into their horizontal and vertical components to obtain real-valued signals. A set number of vectors, between 150 and 800 may be generated. If these vectors are distributed in an equidistant manner in the video frame, their number is determined by a "step" value, which is the size of the pixel neighborhood around which Optical Flow is calculated. This step value lies optimally between 12 and 24, although it may vary depending on desired number of vectors and input resolution. This may generate 192 vectors (16 horizontally, 12 vertically) 240 vectors (20 horizontally, 12 vertically), up to 768 vectors (32 by 24), depending on resolution and aspect ratio of the video camera, desired number of vectors, and the computational performance of the computational unit (200). It is worth noting that once these vectors are split into their horizontal and vertical components, double as many time-series signals are generated.

After these time-series signals are computed with Optical Flow, a dimensionality reduction method may be employed, to reduce the number of signals while maintaining a large proportion of the variance in the signals. This may be, for instance, Principal Component Analysis (PCA), or another dimensionality reduction method. Between 10 and 50 principal components are recovered, depending on the amount of the variance of the original signal they account for. This number may be set a priori or calculated from the data.

Following this step, it is essential to separate the movement corresponding to the occurrence of interest to movement from other sources, such as other people in the video frame. For this purpose, Blind Source Separation (BSS) methods such as Independent Component Analysis (ICA) are performed, using the horizontal and vertical components of the Optical Flow vector signals as observations to estimate the hidden independent sources of movement, separating the movement performed by the user and the movement performed by objects and/or people. This is particularly advantageous when compared to seizure detection methods and devices in the state of the art, in that this device enables contactless seizure detection while isolating movement from the user. The isolation of motion with characteristics specific to the occurrence based on independence enables this method to be particularly robust to extraneous movement sources, enabling it to detect seizures during the realization of physical activities or when other people are in the video frame. Additionally, in situations where methods in the state of the art based on video would produce false detections, such as the movement of other people in the video frame, this device would not, due to the separation of independent components. Additionally, as already mentioned, the processing of movements using time-series signals preserves the user's privacy by design, as these signals cannot be used to identify the user in any way, unlike raw video footage.

To determine which independent components (or sources) correspond to a certain occurrence or extraneous movement, a source selection step is implemented, wherein custom metrics are employed, depending on the occurrence of interest. This may be, for example, a measure for temporal consistency in the case of the detection of tonic-clonic seizures, a peakedness measure in the case of myoclonic seizures, or other metrics for different occurrence types, which may or may not be seizures.

For the case of tonic-clonic seizure detection, a novel temporal consistency metric may be employed. This metric, called the Temporal Consistency Factor (TCF), is the product of two novel metrics, the "Peak to Squared Sum of Roots Ratio" (P2SSR) and the "Percentage of Low and High Amplitude Values" (L/H%). The combination of these two metrics enables the differentiation between seizure and non-seizure movement, which allows the method now developed to specifically select only ICA sources with seizure movement.

P2SSR is the ratio between the maximum value of the signal and the squared sum of the square root of every sample in the signal (SSR), multiplied between a normalization term to ensure that it is only a comparative measure. It can be defined mathematically as follows: $P 2 SSR = normalization term * max \left(\left|s\left(t\right)\right|\right) / SSR \left(s\left(t\right)\right) ,$ Wherein, $SSR = {\left(sum\left(sqrt\left(s\left(t\right)\right)\right)\right)}^{2} ,$ and
s(t) being the signal corresponding to an ICA source, that is independent components associated to user's features movements.

L/H% is the percentage of samples in a signal that lie in a median level of amplitude, having a higher absolute amplitude than a lower threshold and lower absolute amplitude than a higher threshold. The threshold values are programed as a function of an initial training dataset. For example, the lower and the higher thresholds may be defined as 20% of the lower and of the higher absolute amplitude values of the dataset, respectively. The L/H% parameter is a measure for how consistent the signal is over time, which is a characteristic of clonic seizure movement.

TCF is then defined as: $TCF = P 2 SSR * L / H % .$

This source selection step ensures that the device's mode of functioning is versatile to detect different seizures or other occurrences and may be personalized for each individual.

The dimensionality reduction and source separation steps may be used to further segment the analyzed time periods. If, for instance, the time period in which Optical Flow is calculated is 45 seconds, these 45 seconds may be split in 5 smaller 9 second segments, for which the dimensionality reduction and source separation steps can be calculated, to analyze more closely whether a possible detected occurrence has occurred during the entirety of the 45 second segments or solely in a smaller segment of this period. Selecting a smaller segment in this manner may generate more accurate features for the next step, which is classification using Machine Learning techniques. Additionally, this segmentation enables the device to be employed in a clinical setting, in video-EEG sessions, to automatically segment and isolate seizure episodes, facilitating the sometimes arduous process of seizure annotation.

As mentioned, the last step in the detection pipeline concerns classification. This step separates false positives from true positives according to selected features. These features may be adapted for the detection of specific occurrences or in a user-by-user basis. For example, in the case of the detection of tonic-clonic seizures, these features may be based on the area that the movement of interest occupies within the video frame and the frequency of the movement. Simple thresholding or nearest neighbor methods may be employed, as well as more complex machine learning classifiers, such as for example a Support Vector Machine (SVM) classifier, a neural network such as a Multilayer Perceptron (MLP) classifier, or a Gaussian Process Classifier (GPC).

Once an occurrence is detected, the user may select an intended functionality. This may be an automated alarm which may be a mobile notification, audio signal automated phone call or other type of alarm, and may be transmitted to a caregiver, health professional, the emergency services, or another entity. The intended functionality may also consist of saving a video file or merely logging statistical quantitative or qualitative information about the occurrence.

The device now developed delivers various information about the occurrence, including but not limited to the length of the occurrence, the time at which it started and ended, the degree of confidence in the estimation, the video footage of the occurrence, the frequency at which occurrences are detected, the type of occurrence, and other important information.

The primary data acquisition modality is a video camera unit (500). Other modalities may be added to compliment the devices functionality or improve its detection performance, such as, including but not limited to accelerometry, electrocardiography, electromyography, electrodermal activity, electroencephalography, or others. The video camera unit (500) must be compatible with the computational unit (200). It must be able to continually record video footage in an adequate resolution and frame rate. The resolution may be between 80 and 1080 vertical pixels and between 80 and 2000 horizontal pixels. The frame rate may be between 12 and 40 frames per second. A wide-angle camera may be used but is not required. The video camera unit (500) must be able to record night-time footage. For this purpose, it must not have a permanent filter that cuts infrared (IR) wavelengths of light. In an embodiment, this camera may possess an automatically adjustable IR-cut filter, with a light sensor that detects whether lighting conditions are poor, requiring infrared lighting. If so, this IR-cut filter would be automatically disabled and the infrared lighting module (602) would be enabled, ensuring that night-time operation is possible. Moreover, the video camera unit (500) or its lens may be able to automatically orient itself to keep the user fully in frame. This may be achieved with servomotors or by other means.

Depending on the will of the user, the video feed obtained from the video camera unit (500) may be saved and stored continuously, saved only when an occurrence is detected, or not saved at all, with only the Optical Flow vectors for the latest time periods being stored for the purposes of detection.

The enclosure (100) must, as mentioned, allow the device to be as unnoticeable and unobtrusive as possible. It may also enable the device to be powered, in the case of a light-fixture enclosure. In such an embodiment, the power cable for the light fixture would power all components inside the enclosure (100), including the regular and IR lighting modules (601 and 602), the computational unit (200), the video camera unit (500), and the control and transmission modules (300 and 400). Not all of these modules may require power, and some may be powered by the computational unit (200). In a similar embodiment, in which the enclosure is a light bulb, the computational unit (200) may be powered through the light bulb's socket. In such an embodiment, and possibly in others, an AC/DC converter may be included, to transform the AC power from the power grid into DC power that will be used to power the components. In both of these embodiments, the regular and infrared lighting modules (601 and 602) may be integrated into the light bulb itself, ensuring that this light fixture or light bulb would function as both a regular light bulb and an infrared light bulb, delivering favorable lighting conditions for the operation of the video camera unit (500) and ensuring further integration into the subject's home environment. In other embodiments, this enclosure may be integrated into other household objects or encasings, with the caveat that unobtrusiveness must be maintained.

The control module (300) allows the user or a caregiver to interact with or receive information from the device. Possible manifestations of this control module (300) include, but are not limited to a mobile application, a regular or touch screen display, tactile buttons, hand gestures, noise alarms, remote devices with a video feed and speakers similar to a baby monitor, and others. It is important that this control module (300) provides important information to the user or caregiver, such as the time, length, and frequency of seizures or occurrences of interest as well as other statistical information, as well as possibly a live video feed or an automated alarm in the case of an occurrence being detected, and the current mode of functioning of the device (whether it is currently recording, what type of seizure or occurrence it is currently detecting, etc.).

Beyond this, the control module (300) may also enable manual control over the function of the device. This includes but is not limited to: controlling whether the device is currently recording (starting and stopping the recording); controlling the type of occurrence it is detecting; controlling what video or statistical data it is storing locally or remotely; enabling or disabling sharing information with health professionals or emergency services; enabling or disabling automated alarms; and others.

One way of interacting with the device is by a touch screen, enabling a live visualization of the video feed, as well as control of the aforementioned recording parameters. This touch screen can be capacitive or resistive and can possess multi-touch functionality. Any sort of included display can be integrated within the enclosure or remotely. In an embodiment where the display is included in the enclosure, a mechanism can be included to retract and hide the display, so it is not noticeable, to ensure unobtrusiveness. In an embodiment where a display is available remotely, such as in a device similar to a baby monitor, it is placed in a separate room with a caregiver and display a live video feed, so that any false alarms that for instance occur at night can be investigated by the caregiver by simply glancing at the display, instead of having to go to the user's room. Such a device could also include part of the computational unit (200) or transmission module (400). In another embodiment, the control module (300) is comprised by tactile buttons. These buttons can integrate various functionalities, such as starting or stopping the recording, turning the device on or off, activating or deactivating the regular and infrared lighting modules (601 and 602), or other functionalities. These buttons can be placed directly on the enclosure (100) and connected to GPIO pins in a computational unit (200) or in an external device such as the one previously mentioned.

Another embodiment of the control module (400) would be a mobile application. This enables visualization of the live video feed, as well as the implementation of all aforementioned control and information functionalities. This application can also enable seizure logging, contain a seizure diary, and ensure that explicit consent is given when sharing information with health professionals or emergency services.

The transmission module (400) may be any device or system by which the device can transmit data from the computational unit (200) to a local or remote storage location, to the user, to health professionals or to emergency services; or transmit information or data between two or more devices which together constitute the computational unit (200). As mentioned, the data to be transmitted may consist of video footage, Optical Flow vectors, or information, such as information about detected occurrences or statistical data. Data may or may not be stored locally or remotely, with user permission and/or choice. The user may choose to store continuous video footage, only video footage of detected occurrences, or no video footage whatsoever. Various communication protocols may be used to transmit data, such as Wi-fi communications, Bluetooth, Near-Field Communication (NFC), Power-line Communication (PLC), or other methods of wired or wireless data transfer. In an embodiment, the computational unit (200) may connect to the user's Wi-Fi network and securely transfer data to a remote database via the Secure Shell File Transfer Protocol (SFTP), which the user can access through a mobile application, desktop application, web app or website. In another embodiment, a Power-line Communication chip may transmit data through the electrical grid, where a second computational unit or Wi-Fi transmitter may send the information or data to the user via Wi-Fi.

### DESCRIPTION OF FIGURES

To better understand the invention and some of the preferred embodiments, the attached drawings may be analyzed.

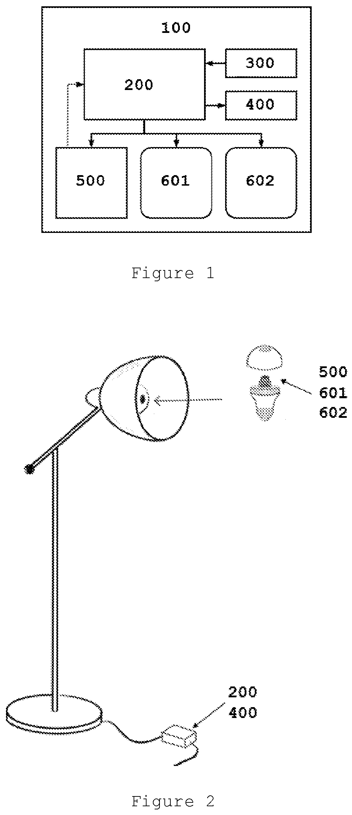

Figure 1 shows the block diagram that illustrates the functional and structural components of the device. An unobtrusive enclosure (100), which ensures the device is unnoticeable and integrated into its surroundings, encloses a computational unit (200), which receives a video signal from the video camera module (500), processes it by executing a privacy-preserving method to detect possible occurrences of interest such as seizures, and transmits this data to the user, caregiver, health professional or the emergency services via the transmission module (400). Regular and infrared lighting modules (601 and 602) ensure that adequate lighting conditions are present. The control module (300) enables the user to manually control the recording, transmission, and functioning process.
Figure 2 shows a preferred embodiment of the device. The device is discreetly integrated into a floor lamp, whereby the video camera unit (500) is integrated into the light bulb, which also contains the regular and infrared lighting modules (601 and 602) in the form of, for example, Light Emitting Diodes integrated into a PCB inside a removable and hot-swappable part of the light bulb. The orientation of the video camera unit (500) may be automatically oriented towards the user using servomotors or by other means. The computational unit (200) is integrated in the AC adaptor power brick included in the power cable of the device. The computational unit may be composed of a Raspberry Pi 4 device or an analogous single-board computer that is capable of implementing the method now developed to detect possible occurrences. The transmission module (400) is included in this device, as the integrated Wi-Fi or Bluetooth communication features of the Raspberry Pi are used to transmit the acquired data via SFTP or another secure data transfer protocol to a remote database or a local device such as the user's phone or computer.

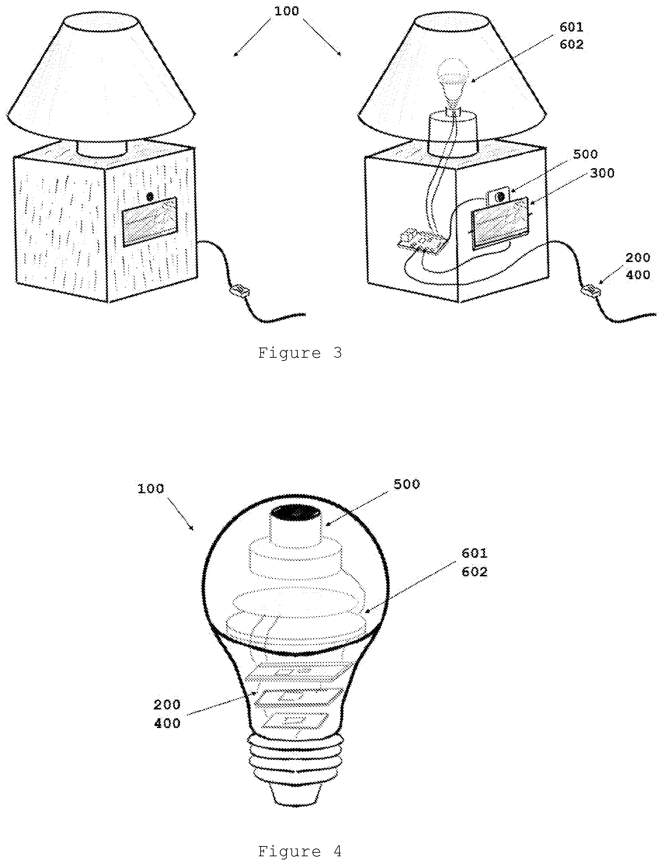

Figure 3 illustrate a preferred embodiment of the device as well as a schematic view of the components inside. In this embodiment, the enclosure (100) is a bedside or desk lamp, and the video camera unit (500) is not placed in the light bulb, but instead discreetly on the side of the support. The orientation of the video camera unit (500) may be automatically oriented towards the user using servomotors or by other means. Beside the camera (500), a capacitive touch screen display serving as the control module (300) is included. This display (300) is placed in a rotatory retractable mechanism, with an axle on either side. This enables it to rotate such that it faces the inside of the enclosure (100), and the back of the display (which is of the same material as the enclosure) is flush with the enclosure (100), hiding the display and ensuring that the device looks as if it is a normal lamp. The material of the enclosure (100) must be dark, such as dark wood or ceramic, in order to hide the camera (500). The light bulb in this enclosure (100) contains regular and infrared LEDs (601 & 602), which are connected to a single-board computer (200) placed inside the enclosure. This computational unit (200) also receives and processes the video input from the camera (500) to detect occurrences, displays it via the included display (300), and transmits data regarding occurrences via wireless communication methods such as Wi-Fi or Bluetooth if the user so desires.
Figure 4 illustrates schematic view of a preferred embodiment of the device, in which the device is enclosed into a light bulb. This enclosure (100) contains the components that are regularly present in a light bulb, such as a diffuser, an enclosure and plate with a thermal conducting material (such as aluminium) coating to transfer heat away from the LEDs (in this case both regular and IR LEDs (601 & 602) in a Printed Circuit Board (PCB))**,** as well as an LED driver circuit with an AC/DC adapter. Besides these components, a small form factor video camera (500) is connected to a small microcontroller (200) such as an Arduino, ESP32 or Raspberry Pi Pico or Zero device, which receives input from the camera (500), computes Optical Flow, and sends it to a Power-Line Communications (PLC) chip (400), which sends the data via the electrical current to a receiver placed on a power outlet. The orientation of the video camera module (500) may be automatically oriented towards the user using servomotors or by other means.

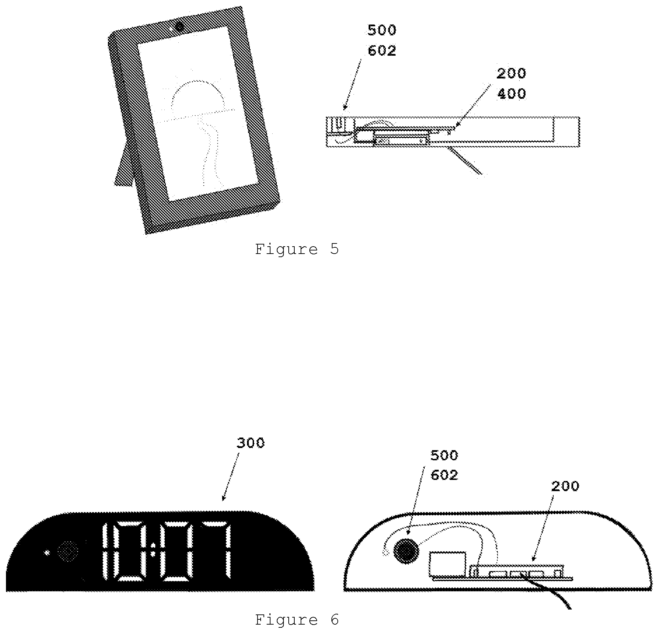

Figure 5 illustrate a preferred embodiment of the device inside a picture frame as well as a schematic view of the components inside. A small form factor video camera (500) is placed on the frame, beside a small infrared LED (602), for operation in low light conditions. Inside the frame, a single-board computer (200) receives input from the video camera (500), processes it, executes the detection method, and transmits it via wireless communications (400) (such as Wi-Fi, Bluetooth, or others). An expansion board (such as a Raspberry Pi HAT) to enable removable batteries to power the device is connected to the single-board computer. This way, the device can be powered by removable batteries, eschewing the need for cables.
Figure 6 illustrate a preferred embodiment of the device inside a bedside digital alarm clock, as well as a schematic view of the components inside. These components are a video camera (500), an IR LED (602) for night-time operation, a single-board computer (200) and a 7-segment display used to display the time. The single-board computer (200) receives video footage from the camera (500), processes it and executes the detection method, and sends data and occurrences detected via Wi-Fi, Bluetooth or other wireless communication methods to a remote database or the user's mobile device, such that it can be displayed in a mobile application (300) which can also control the recording.

### EMBODIMENTS

In a preferred embodiment of the device, the device is discreetly integrated into a floor lamp, whereby the video camera unit (500) is integrated into the light bulb, which also contains the regular and infrared lighting modules (601 and 602) in the form of Light Emitting Diodes integrated into a PCB inside a removable and hot-swappable part of the light bulb. While the light bulb casing itself is not removable, as it contains the video camera unit (500) and the cables of the camera pass through it, this top part which contains the lighting modules is removable (with a screwing method) in order to replace these modules if they fail over time, ensuring that the device has a long lifetime.

The orientation of the video camera unit (500) may be automatically oriented towards the user using servomotors or by other means. The computational unit (200) is integrated in the AC adaptor power brick included in the power cable of the device. The computational unit is composed of a Raspberry Pi 4 device or an analogous single-board computer that is capable of implementing the method now developed to detect possible occurrences. The transmission module (400) is included in this device, as the integrated Wi-Fi or Bluetooth communication features of the Raspberry Pi are used to transmit the acquired data via SFTP or another secure data transfer protocol to a remote database or a local device such as the user's phone or computer. The data may be accessed by the user via a mobile application, which contains the control module (300), enabling the user to control the recording, the lighting modules (601 and 602), the data being recorded, and other parameters.

In another preferred embodiment of the device, the enclosure (100) is a bedside or desk lamp, and the video camera unit (500) is not placed in the light bulb, but instead discreetly on the side of the support. The orientation of the video camera unit (500) may be automatically oriented towards the user using servomotors or by other means. Beside the camera, a capacitive touch screen display serving as the control module (300) is included, allowing the user to view a live video feed of the recording and information regarding time, length, and frequency of seizures or occurrences of interest as well as other statistical information. It may also enable the user to control the recording parameters, including whether the device is currently recording, the type of occurrence it is detecting, what video or statistical data it is storing locally or remotely, enabling or disabling sharing information with health professionals or emergency services, enabling or disabling automated alarms, and others. This display is placed in a rotatory retractable mechanism, with an axle on either side. This enables it to rotate such that it faces the inside of the enclosure (100), and the back of the display (which is of the same material as the enclosure) is flush with the enclosure (100), hiding the display and ensuring that the device looks as if it is a normal lamp. The material of the enclosure is preferably dark, such as dark wood or ceramic, in order to hide the camera (500). The light bulb in this enclosure contains regular and infrared LEDs (601 & 602), which are connected to a single-board computer (200) placed inside the enclosure (100). This computational unit (200) also receives and processes the video input from the camera (500) to detect occurrences, displays it via the included display, and transmits data regarding occurrences via wireless communication methods such as Wi-Fi or Bluetooth if the user so desires.

In another preferred embodiment of the device, the device is enclosed into a light bulb. This enclosure (100) contains the components that are regularly present in a light bulb, such as a diffuser, an enclosure and plate with a thermal conducting material (such as aluminium) coating to transfer heat away from the LEDs (in this case both regular and IR LEDs (601 & 602) in a Printed Circuit Board (PCB)), as well as an LED driver circuit with an AC/DC adapter. Besides these components, a small form factor video camera (500) is connected to a small microcontroller (200) such as an Arduino, ESP32 or Raspberry Pi Pico or Zero device, which receives input from the camera, computes Optical Flow, and sends it to a Power-Line Communications (PLC) chip (400), which sends the data via the electrical current to a receiver placed on a power outlet. This receiver transmits the data to the user's computer or mobile phone via Wi-Fi or another type of wired or wireless communication (such as Bluetooth, USB, or others), which processes the Optical Flow data (which is an unidentifiable representation of the movement data, preserving privacy by design), and executes the detection method. This embodiment has the advantage of being very compact and able to be placed in any lamp of light fixture the user already has, reducing cost. Moreover, the use of a mobile phone or computer to process the data further reduces cost. The orientation of the video camera unit (500) may be automatically oriented towards the user using servomotors or by other means.

A further preferred embodiment of the device encloses it inside a picture frame (100). A small form factor video camera (500) is placed on the frame, beside a small infrared LED (602), for operation in low light conditions. Inside the frame, a single-board computer (200) receives input from the video camera (500), processes it, executes the detection method, and transmits it via wireless communications (400) (such as Wi-Fi, Bluetooth, or others). An expansion board (such as a Raspberry Pi HAT) to enable removable batteries to power the device is connected to the single-board computer. This way, the device can be powered by removable batteries, eschewing the need for cables.

In another preferred embodiment of the device, its placed inside a bedside digital alarm clock (100). Inside it are a video camera unit (500), an IR LED (602) for night-time operation, a single-board computer (200) and a 7-segment display used to display the time. The single-board computer (200) receives video footage from the camera (500), processes it and executes the detection method, and sends data and occurrences detected via Wi-Fi, Bluetooth or other wireless communication methods to a remote database or the user's mobile device, such that it can be displayed in a mobile application (300) which can also control the recording.

Besides embodiments for ambulatory use, this device may also be used for video-EEG acquisition in a hospital setting. In such an embodiment, the video camera (500) with an automatically adjustable lens or body using servomotors or other methods, could be placed on the wall of the video-EEG room. In lieu of a discrete computational unit (200), the computer placed in the video-EEG room may be used to execute the method. In cases where the video-EEG room does not already use a computer, a single-board computer may be used, which would be more affordable than a full-size computer. The seizure detection method which underlies the function of the device would be very advantageous in a video-EEG setting, as it may help to: detect seizures when medical professionals are not present; issue automated alarms to health professionals in the hospital; automatically register time of occurrence, length, type of occurrence, as well as other data; automatically isolate and segment video footage regarding occurrence; and other benefits.

### REFERENCES

[1] T. Herrera-Fortin, E. B. Assi, M.-P. Gagnon, and D. K. Nguyen. Seizure detection devices: A survey of needs and preferences of patients and caregivers. *Epilepsy Behaviour,* Jan 2021.
[2] A. U.-C. et al. Automated seizure detection systems and their effectiveness for each type of seizure. Seizure, Aug 2016.
[3] K. et al. Automatic segmentation of episodes containing epileptic clonic seizures in video sequences. IEEE Transactions on Neural Systems and Rehabilitation Engineering, 59(12):3379- 3385, Dec 2012.
[4] N. B. K. et al. Automated detection of videotaped neonatal seizures based on motion segmentation methods. Clinical Neurophysiology, 117(7):1585-1594, Jul 2006.
[5] Y. Y. et al. Video-based detection of generalized tonic- clonic seizures using deep learning. IEEE Journal of Biomedical And Health Informatics, 25(8):2997-3008, Aug 2021.
[6] H. Plácido da Silva. Biomedical Sensors as Invisible Doctors, pages 322-329. Jan 2019.
[7] Z. K. et al. Passive monitoring at home: A pilot study in parkinson disease. Digital Biomarkers, 3 (1):22-30, Apr. 2019.
[8] Epilepsy: a public health imperative. Technical report, World Health Organization, Geneva, 2019.

## Claims

1. Unobtrusive device for seizure detection comprising:
- an enclosure (100) adapted to enclose:
- a computational unit (200) comprising a control module (300) and a transmission module (400);
- a video camera unit (500);
- a lighting unit comprising a regular (601) and an infrared (602) lighting modules;
wherein
the video camera unit (500) is configured to record a video signal;
the computational unit (200) further comprises a processing module configured to execute the following steps in succession:
i. processing at least a part of the recorded video signal by executing a motion detection step in order to calculate motion features signals;
ii. generating time-series signals from the motion features signals calculated; said motion time-series signals comprising a set of movement vectors;
iii. splitting each movement vectors into an horizontal and a vertical component to obtain a set of real-valued signals;
iv. applying a component separation step by a blind-source separation algorithm, preferably by an Independent Component Analysis algorithm, to the horizontal and vertical components of the real-valued signals to estimate independent components of movement in order to separate feature movements associated to a user from feature movements not associated to a user;
v. executing a source selection step to identify which independent components associated to user's feature movements corresponds to a seizure occurrence;
vi. implementing a classification step to the user's feature movements associated to a seizure occurrence in order to separate false positives from true positives;
vii. executing a functionality in case of a seizure occurrence is detected.

2. Device according to claim 1, wherein said motion features signals relate to movement velocities at equidistant and/or feature-based points in the video signal recorded by the video camera unit (500).

3. Device according to claims 1 or 2, wherein the processing module is configured to implement the motion detection step by an Optical Flow-based algorithm; preferably, the Optical Flow-based algorithm being a Farnebäck two-frame motion estimation method.

4. Device according to any of the previous claims, wherein the processing module is configured to execute a dimensionality reduction step to reduce the number of real-valued signals obtained from splitting movement vectors into horizontal and vertical components.

5. Device according to claim 4, wherein the processing module is configured to implement the dimensionality reduction step by a Principal Component Analysis algorithm; and wherein, between 10 to 50 principal components are recovered.

6. Device according to any of the previous claims wherein the processing module is configured to execute the source selection step by employing a set of metrics to identify which independent components associated to user's feature movements corresponds to a seizure occurrence; said metrics being related to but not limited to:
- temporal consistency for the detection of tonic-clonic seizures; and/or
- peakedness measure for the detection of myoclonic seizures.

7. Device according to claim 6, wherein the temporal consistency metric is a temporal consistency factor (TCF) defined by: $TCF = P 2 SSR * L / H % ,$ Wherein,
P2SSR is the ratio between a maximum value of a signal corresponding to independent components associated to user's feature movements and the squared sum of the square root of every sample in said signal, multiplied between a normalization term;
and
L/H% is the percentage of samples of said signal that lie in a median level of amplitude, having a higher absolute amplitude than a lower threshold and lower absolute amplitude than a higher threshold.

8. Device according to any of the previous claims, wherein the processing module is configured to implement the classification step using thresholding or nearest neighbor algorithms or using a machine learning classifier.

9. Device according to any of the previous claims wherein the computational unit (200) is configured to execute a functionality further involving the steps of issuing an automated alarm and/or saving a video file of the occurrence and/or logging statistical quantitative or qualitative information about the occurrence.

10. Device according to any of the previous claims wherein video camera unit (500) is configured to record the video signal at regular time intervals, between 5 seconds and 2 minutes.

11. Device according to any of the previous claims, wherein the processing module is divided into at least two processing submodules being a first processing submodule within the enclosure (100) and at least a second processing submodule being an external device.

12. Device according to claim 11, wherein the external device is a computer or a smartphone.

13. Device according to any of the previous claims 10 to 12 wherein the video camera unit (500) is adapted to record night-time video signals; and
the resolution of the video camera unit (500) is between 120 and 1080 vertical pixels and between 200 and 2000 horizontal pixels; and
the frame rate of the video camera unit (500) is between 12 and 40 frames per second.

14. Device according to any of the previous claims 10 to 13, wherein the enclosure (100) is a household object, particularly, the enclosure (100) is a light fixture such as a floor lamp or a desk lamp.

## Patentansprüche

1. Unauffällige Vorrichtung zur Anfallserkennung, umfassend:
- ein Gehäuse (100), das dazu ausgelegt ist, Folgendes zu umschließen:
- eine Recheneinheit (200), die ein Steuermodul (300) und ein Übertragungsmodul (400) umfasst;
- eine Videokameraeinheit (500);
- eine Beleuchtungseinheit, die ein normales (601) und ein Infrarot- (602) Beleuchtungsmodul umfasst;
wobei
die Videokameraeinheit (500) so konfiguriert ist, dass sie ein Videosignal aufzeichnet;
die Recheneinheit (200) ferner ein Verarbeitungsmodul umfasst, das so konfiguriert ist, dass es die folgenden Schritte nacheinander ausführt:
i. Verarbeiten mindestens eines Teils des aufgezeichneten Videosignals durch Ausführen eines Bewegungserkennungsschritts, um Bewegungsmerkmalsignale zu berechnen;
ii. Erzeugen von Zeitreihensignalen aus den berechneten Bewegungsmerkmalsignalen; wobei die Bewegungszeitreihensignale einen Satz von Bewegungsvektoren umfassen;
iii. Aufteilen jedes Bewegungsvektors in eine horizontale und eine vertikale Komponente, um eine Menge reellwertiger Signale zu erhalten;
iv. Anwenden eines Komponententrennungsschritts mittels eines Blindquellen-Trennungsalgorithmus, vorzugsweise mittels eines Algorithmus zur unabhängigen Komponentenanalyse, auf die horizontalen und vertikalen Komponenten der reellwertigen Signale, um unabhängige Bewegungskomponenten zu schätzen, damit Merkmalsbewegungen, die einem Benutzer zugeordnet sind, von Merkmalsbewegungen getrennt werden, die keinem Benutzer zugeordnet sind;
v. Ausführen eines Quellenauswahlschritts, um zu identifizieren, welche unabhängigen Komponenten, die mit den Merkmalsbewegungen des Benutzers assoziiert sind, einem Anfallsereignis entsprechen;
vi. Durchführung eines Klassifizierungsschritts für die mit einem Anfallsereignis verbundenen Merkmalsbewegungen des Benutzers, um falsch-positive Ergebnisse von echt-positiven Ergebnissen zu trennen;
vii. Ausführen einer Funktionalität, falls ein Anfallsereignis erkannt wird.

2. Vorrichtung nach Anspruch 1, wobei sich die Bewegungsmerkmalsignale auf Bewegungsgeschwindigkeiten an äquidistanten und/oder merkmalsbasierten Punkten in dem von der Videokameraeinheit (500) aufgezeichneten Videosignal beziehen.

3. Vorrichtung nach Anspruch 1 oder 2, wobei das Verarbeitungsmodul so konfiguriert ist, dass es den Bewegungserkennungsschritt mittels eines auf dem optischen Fluss basierenden Algorithmus durchführt; wobei der auf dem optischen Fluss basierende Algorithmus vorzugsweise eine Farnebäck- Bewegungsschätzmethode für zwei Bilder ist.

4. Vorrichtung nach einem der vorstehenden Ansprüche, wobei das Verarbeitungsmodul so konfiguriert ist, dass es einen Schritt zur Dimensionsreduktion ausführt, um die Anzahl der reellwertigen Signale zu verringern, die durch Aufteilung von Bewegungsvektoren in horizontale und vertikale Komponenten erhalten werden.

5. Vorrichtung nach Anspruch 4, wobei das Verarbeitungsmodul so konfiguriert ist, dass es den Schritt der Dimensionsreduktion mittels eines Algorithmus zur Hauptkomponentenanalyse durchführt; und wobei zwischen 10 und 50 Hauptkomponenten wiederhergestellt werden.

6. Vorrichtung nach einem der vorstehenden Ansprüche, wobei das Verarbeitungsmodul so konfiguriert ist, dass es den Quellenauswahlschritt unter Verwendung einer Reihe von Metriken ausführt, um zu ermitteln, welche unabhängigen Komponenten, die mit den Bewegungsmerkmalen des Benutzers assoziiert sind, einem Anfallsereignis entsprechen; wobei sich die genannten Metriken unter anderem auf Folgendes beziehen, sind jedoch nicht darauf beschränkt:
- zeitliche Konsistenz zur Erkennung tonisch-klonischer Anfälle; und/oder
- Spitzigkeitsmaß zur Erkennung myoklonischer Anfälle.

7. Vorrichtung nach Anspruch 6, wobei die Metrik der zeitlichen Konsistenz ein Faktor der zeitlichen Konsistenz (TCF) ist, der wie folgt definiert ist: $TCF = P 2 SSR * L / H % ,$ wobei
P2SSR das Verhältnis zwischen einem Maximalwert eines Signals, das unabhängigen Komponenten entspricht, die mit den Bewegungsmerkmalen des Benutzers assoziiert sind, und der quadrierten Summe der Quadratwurzeln aller Abtastwerte in diesem Signal, multipliziert mit einem Normalisierungsfaktor, ist;
und
L/H% der Prozentsatz der Abtastwerte des besagten Signals ist, die in einem mittleren Amplitudenbereich liegen, eine höhere absolute Amplitude als ein unterer Schwellenwert und eine niedrigere absolute Amplitude als ein oberer Schwellenwert aufweisen.

8. Vorrichtung nach einem der vorstehenden Ansprüche, wobei das Verarbeitungsmodul so konfiguriert ist, dass es den Klassifizierungsschritt unter Verwendung von Schwellenwert- oder Next-Nearest-Neighbor-Algorithmen oder unter Verwendung eines Klassifikators für maschinelles Lernen durchführt.

9. Vorrichtung nach einem der vorstehenden Ansprüche, wobei die Recheneinheit (200) so konfiguriert ist, dass sie eine Funktionalität ausführt, die ferner die Schritte des Auslösens eines automatisierten Alarms und/oder des Speicherns einer Videodatei des Ereignisses und/oder des Protokollierens statistischer quantitativer oder qualitativer Informationen über das Ereignis umfasst.

10. Vorrichtung nach einem der vorstehenden Ansprüche, wobei die Videokameraeinheit (500) so konfiguriert ist, dass sie das Videosignal in regelmäßigen Zeitintervallen zwischen 5 Sekunden und 2 Minuten aufzeichnet.

11. Vorrichtung nach einem der vorstehenden Ansprüche, wobei das Verarbeitungsmodul in mindestens zwei Verarbeitungs-Submodule unterteilt ist, nämlich ein erstes Verarbeitungs-Submodul innerhalb des Gehäuses (100) und mindestens ein zweites Verarbeitungs-Submodul, das ein externes Gerät ist.

12. Vorrichtung nach Anspruch 11, wobei das externe Gerät ein Computer oder ein Smartphone ist.

13. Vorrichtung nach einem der vorstehenden Ansprüche 10 bis 12, wobei die Videokameraeinheit (500) dazu ausgelegt ist, nächtliche Videosignale aufzuzeichnen; und
die Auflösung der Videokameraeinheit (500) zwischen 120 und 1080 vertikalen Pixeln und zwischen 200 und 2000 horizontalen Pixeln liegt; und
die Bildrate der Videokameraeinheit (500) zwischen 12 und 40 Bildern pro Sekunde liegt.

14. Vorrichtung nach einem der vorstehenden Ansprüche 10 bis 13, wobei das Gehäuse (100) ein Haushaltsgegenstand ist, insbesondere ist das Gehäuse (100) eine Leuchte, wie beispielsweise eine Stehlampe oder eine Schreibtischlampe.

## Revendications

1. Dispositif discret de détection de crises comprenant :
- un boîtier (100) adapté pour contenir :
- une unité de calcul (200) comprenant un module de commande (300) et un module de transmission (400) ;
- une unité de caméra vidéo (500) ;
- une unité d'éclairage comprenant un module d'éclairage conventionnel (601) et un module d'éclairage infrarouge (602) ;
dans lequel
l'unité de caméra vidéo (500) est configurée pour enregistrer un signal vidéo ;
l'unité de calcul (200) comprend en outre un module de traitement configuré pour exécuter successivement les étapes suivantes :
i. traiter au moins une partie du signal vidéo enregistré par exécution d'une étape de détection de mouvement afin de calculer des signaux de caractéristiques de mouvement ;
ii. générer des signaux de séries temporelles à partir des signaux de caractéristiques de mouvement calculés ; lesdits signaux de séries temporelles de mouvement comprenant un ensemble de vecteurs de mouvement ;
iii. décomposer chaque vecteur de mouvement en une composante horizontale et une composante verticale afin d'obtenir un ensemble de signaux à valeurs réelles ;
iv. appliquer une étape de séparation de composantes au moyen d'un algorithme de séparation aveugle de sources, de préférence au moyen d'un algorithme d'analyse en composantes indépendantes, aux composantes horizontale et verticale des signaux à valeurs réelles afin d'estimer des composantes indépendantes de mouvement, de manière à séparer les caractéristiques de mouvement associées à un utilisateur des caractéristiques de mouvement non associées à un utilisateur ;
v. exécuter une étape de sélection de sources afin d'identifier quelles composantes indépendantes associées aux caractéristiques de mouvement de l'utilisateur correspondent à l'occurrence d'une crise ;
vi. mettre en œuvre une étape de classification des caractéristiques de mouvement de l'utilisateur associées à l'occurrence d'une crise afin de séparer les faux positifs des vrais positifs ;
vii. exécuter une fonctionnalité dans le cas où l'occurrence d'une crise est détectée.

2. Dispositif selon la revendication 1, dans lequel lesdits signaux de caractéristiques de mouvement se rapportent à des vitesses de mouvement à des points équidistants et/ou des points basés sur des caractéristiques dans le signal vidéo registré par l'unité de caméra vidéo (500).

3. Dispositif selon les revendications 1 ou 2, dans lequel le module de traitement est configuré pour mettre en œuvre l'étape de détection de mouvement au moyen d'un algorithme basé sur le flux optique ; de préférence, l'algorithme basé sur le flux optique étant une méthode d'estimation du mouvement entre deux images de Farnebäck.

4. Dispositif selon l'une quelconque des revendications précédentes, dans lequel le module de traitement est configuré pour exécuter une étape de réduction de dimensionnalité afin de réduire le nombre de signaux à valeurs réelles obtenus par décomposition des vecteurs de mouvement en composantes horizontale et verticale.

5. Dispositif selon la revendication 4, dans lequel le module de traitement est configuré pour mettre en œuvre l'étape de réduction de dimensionnalité au moyen d'un algorithme d'analyse en composantes principales ; et dans lequel sont récupérées entre 10 et 50 composantes principales.

6. Dispositif selon l'une quelconque des revendications précédentes, dans lequel le module de traitement est configuré pour exécuter l'étape de sélection de sources en utilisant un ensemble de métriques afin d'identifier quelles composantes indépendantes associées aux caractéristiques de mouvement de l'utilisateur correspondent à l'occurrence d'une crise ; lesdites métriques se rapportant mais ne se limitant pas à :
- la cohérence temporelle pour la détection des crises tonico-cloniques ; et/ou
- la mesure de la netteté des pics pour la détection des crises myocloniques.

7. Dispositif selon la revendication 6, dans lequel la métrique de cohérence temporelle est un facteur de cohérence temporelle (FCT) défini par : $TCF = P 2 SSR * L / H % ,$ où
P2SSR est le rapport entre une valeur maximale d'un signal correspondant à des composantes indépendantes associées aux caractéristiques de mouvement de l'utilisateur et le carré de la somme de la racine carrée de chaque échantillon dudit signal, multiplié par un terme de normalisation
et
L/H% est le pourcentage d'échantillons dudit signal qui se situent à un niveau médian d'amplitude, ayant une amplitude absolue supérieure à un seuil inférieur et une amplitude absolue inférieure à un seuil supérieur.

8. Dispositif selon l'une quelconque des revendications précédentes, dans lequel le module de traitement est configuré pour mettre en œuvre l'étape de classification au moyen d'algorithmes de seuillage ou des plus proches voisins, ou en utilisant un classificateur d'apprentissage automatique.

9. Dispositif selon l'une quelconque des revendications précédentes, dans lequel l'unité de calcul (200) est configurée pour exécuter une fonctionnalité comprenant en outre les étapes d'émettre une alarme automatique et/ou d'enregistrer un fichier vidéo de l'occurrence et/ou de consigner des informations statistiques quantitatives ou qualitatives concernant l'occurrence.

10. Dispositif selon l'une quelconque des revendications précédentes, dans lequel l'unité de caméra vidéo (500) est configurée pour enregistrer le signal vidéo à des intervalles de temps réguliers, entre 5 secondes et 2 minutes.

11. Dispositif selon l'une quelconque des revendications précédentes, dans lequel le module de traitement est divisé en au moins deux sous-modules de traitement, un premier sous-module de traitement étant situé à l'intérieur du boîtier (100) et au moins un deuxième sous-module de traitement étant un dispositif externe.

12. Dispositif selon la revendication 11, dans lequel le dispositif externe est un ordinateur ou un smartphone.

13. Dispositif selon l'une quelconque des revendications précédentes 10 à 12, dans lequel l'unité de caméra vidéo (500) est adaptée pour enregistrer des signaux de vidéo nocturnes ; et
la résolution de l'unité de caméra vidéo (500) est comprise entre 120 et 1080 pixels verticaux et entre 200 et 2000 pixels horizontaux ; et
la fréquence d'images de l'unité de caméra vidéo (500) est comprise entre 12 et 40 images par seconde.

14. Dispositif selon l'une quelconque des revendications précédentes 10 à 13, dans lequel le boîtier (100) est un objet domestique, en particulier le boîtier (100) est un luminaire tel qu'un lampadaire ou une lampe de bureau.
